(19) Europäisches Patentamt
European Patent Office
Office européen des brevets

(11) Veröffentlichungsnummer: **0 624 587 A2**

# (12) EUROPÄISCHE PATENTANMELDUNG

(21) Anmeldenummer: **94107390.0**

(51) Int. Cl.$^5$: **C07D 491/048**

(22) Anmeldetag: **11.05.94**

(30) Priorität: **14.05.93 CH 1452/93**

(43) Veröffentlichungstag der Anmeldung:
**17.11.94 Patentblatt 94/46**

(84) Benannte Vertragsstaaten:
**AT BE CH DE DK ES FR GB IE IT LI NL PT SE**

(71) Anmelder: **LONZA A.G.**

**CH-3945 Gampel/Wallis (CH)**

(72) Erfinder: **McGarrity, John, Dr.**
**Gemsweg 4**
**Visp (Kanton Wallis) (CH)**
Erfinder: **Spindler, Felix, Dr.**
**Niederamtstrasse 415**
**Starrkirch-Wil (Kanton Soluthurn) (CH)**
Erfinder: **Fuchs, Rudolf, Dr.**
**Rue du Petit-Chasseur 34**
**Sion (Kanton Wallis) (CH)**
Erfinder: **Eyer, Martin, Dr.**
**Hengart 15**
**Brig-Glis (Kanton Wallis) (CH)**

(74) Vertreter: **Weinhold, Peter, Dr. et al**
**Patentanwälte Dipl.-Ing. G. Dannenberg**
**Dr. P. Weinhold**
**Dr. D. Gudel**
**Dipl.-Ing. S. Schubert**
**Dr. P. Barz**
**Siegfriedstrasse 8**
**D-80803 München (DE)**

(54) **Asymmetrische Hydrierung von Furoimidazol-derivaten.**

(57) Es wird ein neues Verfahren zur asymmetrischen Hydrierung von Furoimidazolderivaten der allgemeinen Formel

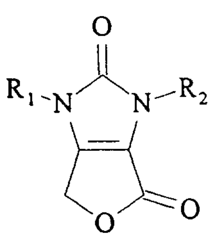

I,

mit Wasserstoff in Gegenwart eines Homogenkatalysators zu den entsprechenden diastereomeren Dihydrofuroimidazolderivaten der allgemeinen Formel

EP 0 624 587 A2

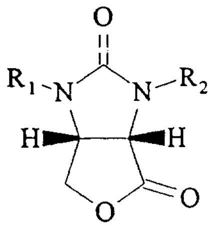

II

beschrieben.
Die Dihydrofuroimidazolderivate der allgemeinen Formel II sind Zwischenprodukte zur Herstellung des Vitamins (+)-Biotin.

Die Erfindung betrifft ein neues Verfahren zur asymmetrischen Hydrierung von Furoimidazolderivaten der allgemeinen Formel

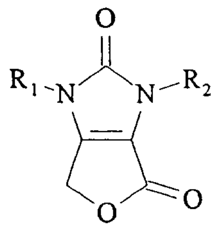

I,

worin $R_1$ eine auf bekannte Weise abspaltbare Schutzgruppe bedeutet und $R_2$ für Wasserstoff oder eine auf bekannte Weise abspaltbare Schutzgruppe steht, mit Wasserstoff in Gegenwart eines Homogenkatalysators zu den entsprechenden diastereomeren Dihydrofuroimidazolderivaten der allgemeinen Formel

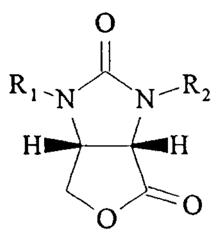

II

worin $R_1$ und $R_2$ die genannte Bedeutung haben.

Die Dihydrofuroimidazole der allgemeinen Formel II sind wichtige Zwischenprodukte in der Synthese von (+)-Biotin, einem für den Menschen essentiellen Vitamin, das auch als Vitamin H bezeichnet wird. (+)-Biotin wird ausserdem als Pharmazeutikum zur Behandlung von Dermatosen oder als Futtermittelzusatz mit wachstumsfördernder Wirkung für Nutztiere eingesetzt.

Die meisten bekannten (+)-Biotin-Synthesen verfolgen das Ziel, geeignete Vorstufen über zum Teil sehr aufwendige Racematspaltungsmethoden mit zum Teil sehr teuren Spaltungs-agentien zu trennen und mit den resultierenden Enantiomeren die (+)-Biotinsynthese weiterzuverfolgen (vgl. z. B. DE-PS 2 058 248). Gemäss EP-PS 273 270 erreichte man dann erstmals die Einführung der korrekten Konfiguration, das heisst (S) in der 3a und (R) in der 6a-Position der Biotinringstruktur, über eine asymmetrische Hydrierung entsprechender Furoimidazolderivate mit einem klassischen Hydrierkatalysator wie zum Beispiel Rhodium auf Aluminiumoxid. Bezüglich der erreichbaren Ausbeute am gewünschten Diastereomeren konnte dieses Verfahren jedoch nicht restlos befriedigen.

Es bestand folglich die Aufgabe, ein verbessertes asymmetrisches Hydrierverfahren zu entwickeln, mit welchem man den genannten Schlüsselschritt der Biotinsynthese mit einer sehr guten Diastereoselektivität bei guter Ausbeute des Dihydrofuroimidazols durchführen kann.

Diese Aufgabe konnte gelöst werden mit dem erfindungsgemässen Verfahren nach Anspruch 1.

Die Furoimidazole der allgemeinen Formel I können gemäss den Vorschriften des EP-PS 273 270 oder der EP-PS 270 076 hergestellt werden.

Zweckmässig werden als auf bekannte Weise abspaltbare Schutzgruppen für $R_1$ nachfolgende Gruppen eingesetzt:

Eine 1-Phenyl-($C_1$-$C_6$)-alkylgruppe oder eine 1-Naphthyl-($C_1$-$C_6$)-alkylgruppe. Deren aromatische Kerne können gegebenenfalls mit einem oder mehreren Substituenten aus der Reihe ($C_1$-$C_6$)-Alkyl, ($C_1$-$C_6$)-Alkoxy, Hydroxy, Halogen, Amino, ($C_1$-$C_6$)-Alkylamino, ($C_1$-$C_6$)-Dialkylamino substituiert sein.

Die 1-Phenyl-($C_1$-$C_6$)-alkylgruppe oder die 1-Naphthyl-($C_1$-$C_6$)-alkylgruppe kann ein chirales Zentrum aufweisen.

$R_1$ hat bevorzugt die Bedeutung einer (R)- oder (S)-1-Phenylethylgruppe, einer Benzylgruppe oder einer (R)- oder (S)-1-Naphthylethylgruppe, wobei die aromatischen Kerne der bevorzugten Gruppen mit den genannten Substituenten substituiert sein können.

$R_2$ kann für Wasserstoff oder für auf bekannte Weise abspaltbare Schutzgruppen der Reihe ($C_1$-$C_6$)-Alkanoyl, Benzyl, ($C_1$-$C_6$)-Alkoxy-($C_1$-$C_6$)-alkyl, ($C_1$-$C_6$)-Alkoxycarbonyl oder Aroyl wie z. B. Benzoyl stehen. Der aromatische Kern der Benzylgruppe oder der Aroylgruppen kann den aromatischen Kernen von $R_1$

entsprechend substituiert sein.

$R_2$ hat bevorzugt die Bedeutung von Wasserstoff, Acetyl, Benzyl, $(C_1-C_2)$-Alkoxy-$(C_1-C_2)$-alkyl, $(C_1-C_2)$-Alkoxycarbonyl oder Benzoyl.

Überraschenderweise wurde gefunden, dass Homogenkatalysatoren, die aus einem Rh-Komplex und einem chiralen Phosphin-Liganden gebildet werden, eine hohe Stereoselektivität bei in der Regel guter Ausbeute zeigen.

Als Rh-Komplexe finden solche der allgemeinen Formel

Rh(0): $[Rh\ (L)\ A]_2$ III

oder

Rh(I): $[Rh\ (L)_2]B$ IV

Anwendung, worin L für zwei $C_2$-$C_{12}$ Olefine oder ein $C_5$-$C_{12}$ Dien steht, A ein Halogen und B ein Anion einer Sauerstoffsäure oder Komplexsäure bedeutet.

L in der Bedeutung als Olefin enthält bevorzugt 2 bis 6 C-Atome und in der Bedeutung als Dien bevorzugt 5 - 8 C-Atome. Das Dien kann dabei offenkettig, mono- oder bicyclisch sein. Beispiele für Olefine sind: Ethylen, Propen oder 1-Buten.

Beispiele für Diene sind: 1,5-Hexadien, 1,4-Cyclohexadien, 1,4- oder 1,5-Heptadien, 1,4-Cycloheptadien, 1,4- oder 1,5-Octadien, 1,4- oder 1,5-Cyclooctadien, Norbornadien.

Bevorzugt stellt L zwei Ethylen oder ein 1,5-Hexadien, 1,5-Cyclooctadien oder Norbornadien dar.

A steht bevorzugt für Chlor oder Brom.

Beispiele für B sind: $ClO_4^-$, $FSO_3^-$, $CH_3SO_3^-$, $CF_3SO_3^-$, $BF_4^-$, $PF_6^-$, $SbCl_6^-$, $AsF_6^-$ und $SbF_6^-$.

Bevorzugt bedeutet B: $BF_4^-$, $ClO_4^-$, $PF_6^-$, $CF_3SO_3^-$ und $SbF_6^-$.

Die Herstellung dieser Rh-Komplexe ist bekannt und geht zum Beispiel aus J. Chatt et al., J. Chem. Soc. 1957, 4735 oder aus G. Giordano et al., Inorg. Synth. 1979, 19, 218 hervor.

Als chirale Phosphin-Liganden finden nachstehende Verbindungen Anwendung:

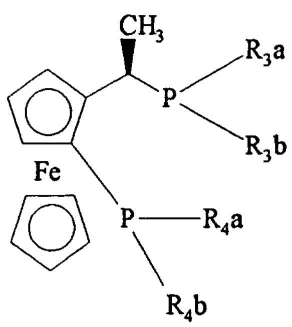

V

$R_{3a}$ und $R_{3b}$ sind gleich oder verschieden und bedeuten $C_1$-$C_{12}$-Alkyl, $C_5$-$C_7$-Cycloalkyl oder $C_6$-$C_{12}$-Aryl; $R_{4a}$ und $R_{4b}$ sind gleich oder verschieden und bedeuten $C_1$-$C_{12}$-Alkyl, $C_5$-$C_7$-Cycloalkyl oder $C_6$-$C_{12}$-Aryl.

Bevorzugt sind $R_{3a}$ und $R_{3b}$ gleich. $R_{3a}$ und $R_{3b}$ in der Bedeutung von Alkyl können linear oder verzweigt sein und bevorzugt 1 bis 6, besonders bevorzugt 1 bis 4 C-Atome enthalten. Bevorzugte Alkylgruppen sind Methyl, Ethyl, Propyl, i-Propyl, n-, i-, und t-Butyl besonders bevorzugt i-Propyl oder t-Butyl. $R_{3a}$ und $R_{3b}$ in der Bedeutung von Cycloalkyl haben bevorzugt die Bedeutung von Cyclohexyl.

$R_{3a}$ und $R_{3b}$ in der Bedeutung von Aryl kann gegebenenfalls substituiertes Phenyl oder Naphthyl bedeuten. Als Substituenten kommen Alkylgruppen oder Alkoxygruppen mit 1 bis 4 C-Atomen oder eine $C_1$-$C_4$-Dialkylaminogruppe in Frage.

$R_{4a}$ und $R_{4b}$ können die für $R_{3a}$ und $R_{3b}$ genannte Bedeutung haben. Besonders bevorzugt steht $R_{4a}$ und $R_{4b}$ für eine Phenylgruppe.

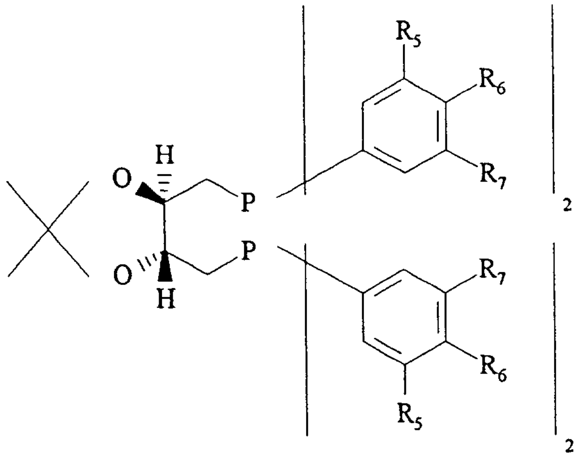

VI

$R_5$ und $R_7$ sind gleich oder verschieden und bedeuten $C_1$-$C_4$-Alkyl; $R_6$ bedeutet $C_1$-$C_4$-Alkoxy oder $C_1$-$C_4$-Dialkylamino.

Bevorzugt sind $R_5$ und $R_7$ gleichbedeutend. In der Bedeutung von Alkyl können $R_5$ und $R_7$ linear oder verzweigt sein. Bevorzugte Alkylgruppe ist die Methylgruppe.

$R_6$ hat bevorzugt die Bedeutung von Methoxy oder Dimethylamino.

Die Phosphinliganden der allgemeinen Formel VI sind literaturbekannt. (T. Morimoto et al. Tetrahedron Letters, 1988, 29, 4755)

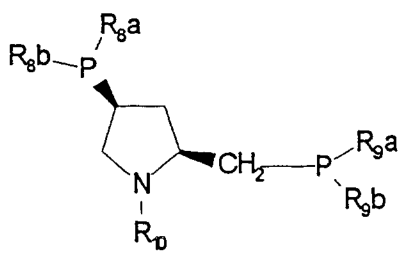

VII

$R_{8a}$ und $R_{8b}$ haben zweckmässig die für $R_{3a}$ und $R_{3b}$ genannte Bedeutung. Bevorzugte Bedeutung von $R_{8a}$ und $R_{8b}$ ist Cyclohexyl oder Phenyl.

$R_{9a}$ und $R_{9b}$ haben zweckmässig die für $R_{3a}$ und $R_{3b}$ angegebene Bedeutung. Bevorzugte Bedeutung ist Phenyl.

$R_{10}$ hat zweckmässig die Bedeutung von Wasserstoff oder einer üblichen $NH_2$-Schutzgruppe. Unter einer übichen $NH_2$-Schutzgruppe werden solche verstanden wie sie in Houben-Weyl, Methoden der org. Chem., Band 15, Thieme Verlag, Stuttgart, 1974 beschrieben sind. Beispielhaft seien die t-Butoxycarbonyl- oder die Benzyloxycarbonylgruppe genannt. Die Verbindungen der allgemeinen Formel VII sind literaturbekannt. (K. Achiwa, J. Am. Chem. Soc. 1976, 98, 8265)

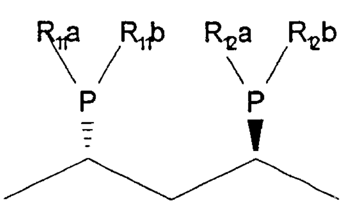

VIII

$R_{11a}$,$R_{11b}$ und $R_{12a}$, $R_{12b}$ haben zweckmässig die für $R_{3a}$ und $R_{3b}$ genannte Bedeutung. Bevorzugt steht $R_{11a}$, $R_{11b}$ und $R_{12a}$, $R_{12b}$ für Phenyl.

Die Verbindungen der allgemeinen Formel VIII sind literaturbekannt (B. Bosnich, J. Am. Chem. Soc. 1981, 103, 2273).

Die Bildung des aktiven Homogenkatalysators erfolgt zweckmässig in situ, das heisst im Rahmen der Hydrierung des betreffenden Furoimidazols der allgemeinen Formel I. Es ist aber ebenso möglich zunächst aus dem Phosphin-Liganden und dem Rh-Komplex einen sogenannten Katalysatorvorläufer (precursor) zu isolieren, welcher separat der Reaktion zugegeben werden kann.

Zweckmässig wird aber so vorgegangen, dass zunächst die Homogenkatalysatorkomponenten, das heisst der Rh-Komplex und der entsprechende Phosphin-Ligand, zusammen mit dem entsprechenden Furoimidazolderivat in einem geeigneten inerten Lösungsmittel vorgelegt werden.

Als Lösungsmittel, die alleine oder im Gemisch angewendet werden können, eignen sich zweckmässig aprotische Lösungsmittel wie z. B. aliphatische oder aromatische Kohlenwasserstoffe oder halogenierte Kohlenwasserstoffe.
Geeignete Vertreter der aromatischen Kohlenwasserstoffe sind z. B. Benzol, Toluol oder Xylol, der aliphatischen Kohlenwasserstoffe z. B. Hexan oder Pentan und der halogenierten Kohlenwasserstoffe Methylenchlorid oder Chloroform. Ein besonders geeignetes Lösungsmittel ist Toluol. Es kann unter Umständen von Vorteil sein, in einer Mischung der genannten Lösungsmittel mit einem protischen Lösungsmittel, wie z. B. mit aliphatischen Alkoholen, zu arbeiten. Ein besonders geeigneter aliphatischer Alkohol ist Methanol.

Die Lösungsmittelmenge wird zweckmässig so gewählt, dass eine Substratkonzentration von 2% bis 20% erhalten wird. Bevorzugt wird bei einer Substratkonzentration von ca. 10% gearbeitet.

Die Katalysatormenge, ausgedrückt als Verhältnis Substrat (Furoimidazol) zu Katalysator, bewegt sich zweckmässig zwischen 100 : 1 und 10'000 : 1, bevorzugt im Bereich zwischen 500 : 1 und 2'000 : 1.

Die Umsetzung erfolgt von Vorteil bei einem Wasserstoffdruck zwischen 1 bar und 200 bar, vorzugsweise 10 bar bis 100 bar, und bei einer Reaktionstemperatur zwischen 25 °C und 150 °C, vorzugsweise 40 °C und 90 °C.

Nach der Hydrierung, die in der Regel 2 bis 6 Stunden dauert, kann das gewünschte stereoisomere (3aS, 6aR)-Dihydrofuroimidazol der allgemeinen Formel II auf fachmännische Weise isoliert werden.

Durch Umkristallisation mit einem geeigneten Lösungsmittel wie zum Beispiel Methylisobutylketon, Ethylacetat oder Toluol können allfällige Anteile des unerwünschten (3aR, 6aS)-Dihydrofuroimidazols entfernt werden.

Die resultierenden Dihydrofuroimidazole können dann zum Beispiel entsprechend der EP-PS 273 270 weiter in das (+)-Biotin umgesetzt werden.

Beispiele:

Die in Tabellenform aufgeführten Hydrierungsbeispiele wurden analog folgender Vorschrift durchgeführt:
In einen 50 ml-Stahlautoklav wurden 0.01 mol des entsprechenden Furoimidazolderivats (Tabelle 1) eingetragen. In einem 10 ml Schlenkgefäss wurden nacheinander 0.001 mmol Rh-Komplex (Tabelle 2) und 0.002 mmol des entsprechenden Phosphin-Liganden (Tabelle 3) in 25 ml Lösungsmittel gelöst und dann während 30 min bei Raumtemperatur gerührt. Diese Katalysatorlösung wurde mittels einer Stahlkapillare in den unter Argonatmosphäre stehenden Autoklaven transferiert, anschliessend wurde der Autoklav während 45 Minuten auf die Temperatur T erwärmt. Nach dem Spülen mit $H_2$ wurde der $H_2$-Druck p aufgepresst. Nach der Reaktionszeit t wurde die Reaktion abgebrochen.
Der Umsatz und die Stereoselektivität (Diastereoselektivität / Enantioselektivität) der erhaltenen Dihydrofuroimidazole wurden mittels $^1$H-NMR (400 MHz) oder mittels HPLC bestimmt.

Tabelle 1

| eingesetzte Furoimidazolderivate | | |
|---|---|---|
| | R1 | R2 |
| A | (R)-1-Phenylethyl | H |
| B | Benzyl | H |
| C | (R)-1-Phenylethyl | Benzyl |
| D | (R)-1-(2-Methoxyphenyl)ethyl | H |
| E | 2-Methoxybenzyl | H |
| F | (R)-1-(1-Naphthyl)ethyl | H |
| G | (R)-1-Phenylethyl | Acetyl |

Tabelle 2

| eingesetzte Rh-Komplexe | |
|---|---|
| Rh(0) | [Rh (norbornadien)Cl]$_2$ |
| Rh(I) | [Rh (norbornadien)$_2$]$BF_4$ |

Tabelle 3

| eingesetzte chirale Phosphin-Liganden | | |
|---|---|---|
| allg. Formel | Definition der Reste | Kurzbezeichnung |
| V(a) | $R_{3a}$, $R_{3b}$ = Cyclohexyl<br>$R_{4a}$, $R_{4b}$ = Phenyl | (R)-(S)-PPF-Pcy2 |
| V(b) | $R_{3a}$, $R_{3b}$ = t-Butyl<br>$R_{4a}$, $R_{4b}$ = Phenyl | (R)-(S)-PPF-PtBu2 |
| VI | $R_5$, $R_7$ = Methyl<br>$R_6$ = Methoxy | (4R, 5R)-MOD-DIOP |
| VII(a) | $R_{8a}$, $R_{8b}$ = Cyclohexyl<br>$R_{9a}$, $R_{9b}$ = Phenyl<br>$R_{10}$ = t-Butoxycarbonyl | (2S, 4S)-BCPM |
| VII(b) | $R_{8a}$, $R_{8b}$ = Phenyl<br>$R_{9a}$, $R_{9b}$ = Phenyl<br>$R_{10}$ = t-Butoxycarbonyl | (2S, 4S)-BPPM |
| VIII | $R_{11a}$, $R_{11b}$ = Phenyl<br>$R_{12a}$, $R_{12b}$ = Phenyl | (2S, 4S)-BDPP |

Tabelle 4

Ausführungsbeispiele:

| Beispiele | Furoimidazol | Rh-Komplex | Ligand | Lösungsmittel | Druck | Temperatur | Reaktionszeit | Umsatz | D'Verhältnis oder E'Verhältnis # |
|---|---|---|---|---|---|---|---|---|---|
| | (Tabelle 1) | Tabelle 2 | (Tabelle 3) | | p (bar) | T (°C) | t (h) | [%] | [%] |
| 1 | A | Rh(0) | VIIb | $CH_2Cl_2$ | 35 | 25 | 48 | 29 | 72 : 28 |
| 2 | A | Rh(0) | V(a) | Tol / MeOH 25 / 3 | 30 | 70 | 28 | 95 | 90 : 10 |
| 3 | A | Rh(0) | VI | Toluol | 35 | 70 | 3 | 100 | 85 : 15 |
| 4 | A | Rh(I) | VI | Tol / MeOH 1 / 1 | 70 | 60 | 19 | 73 | 80 : 20 |
| 5 | A | Rh(I) | VII(a) | THF | 70 | 60 | 43 | 49 | 34 : 66 |
| 6 | A | Rh(0) | V(b) | Toluol | 50 | 70 | 18 | 100 | 99 : 1 |
| 7 | A | Rh(0) | VIII | Tol / MeOH 4 / 1 | 35 | 70 | 19 | 51 | 75 : 25 |
| 8 | C | Rh(0) | VI | Toluol | 30 | 70 | 67 | 9 | 60 : 40 |
| 9 | D | Rh(0) | VI | Toluol | 30 | 70 | 16 | 12 | 92 : 8 |
| 10 | D | Rh(0) | V(b) | Toluol | 50 | 70 | 19 | 72 | 99 : 1 |
| 11 | B | Rh(0) | VI | Toluol | 35 | 70 | 3 | 95 | 95 : 5# |
| 12 | B | Rh(0) | V(b) | Toluol | 50 | 70 | 18 | 95 | 95 : 5# |
| 13 | E | Rh(0) | V(b) | Toluol | 50 | 70 | 18 | 95 | 90 : 10# |
| 14 | F | Rh(0) | VI | Toluol | 30 | 70 | 20 | 97 | 99 : 1 |
| 15 | G | Rh(0) | V(b) | Toluol | 50 | 70 | 69 | 99 | 57 : 43 |

## Patentansprüche

**1.** Verfahren zur asymmetrischen Hydrierung von Furoimidazolderivaten der allgemeinen Formel

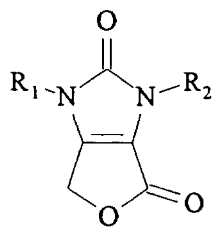

I,

worin $R_1$ eine auf bekannte Weise abspaltbare Schutzgruppe und $R_2$ Wasserstoff oder eine auf bekannte Weise abspaltbare Schutzgruppe bedeutet, mit Wasserstoff in Gegenwart eines Homogenkatalysators zu den entsprechenden diastereomeren Dihydro-furoimidazolderivaten der allgemeinen Formel

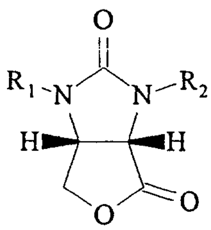

II

worin $R_1$ und $R_2$ die genannte Bedeutung haben, dadurch gekennzeichnet, dass Homogenkatalysatoren eingesetzt werden, die aus einem Rh-Komplex und einem chiralen Phosphin-Liganden ausgewählt aus einer der nachfolgenden allgemeinen Formeln V bis VIII gebildet werden.

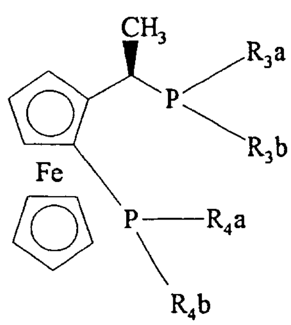

V

worin $R_{3a}$ und $R_{3b}$ gleich oder verschieden sind und $C_1$-$C_{12}$-Alkyl, $C_5$-$C_7$-Cycloalkyl oder $C_6$-$C_{12}$-Aryl bedeuten; $R_{4a}$ und $R_{4b}$ die für $R_{3a}$ und $R_{3b}$ angegebene Bedeutung haben

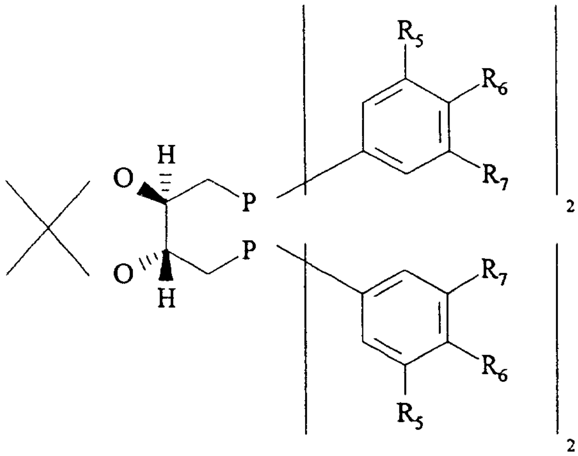

VI

worin $R_5$ und $R_7$ die für $R_{3a}$ und $R_{3b}$ angegebenen Bedeutung haben; $R_6$ $C_1$-$C_4$-Alkoxy oder $C_1$-$C_4$-Dialkylamino bedeutet

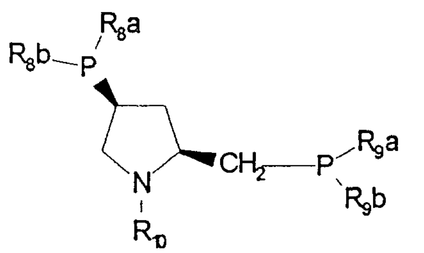

VII

worin $R_{8a}$, $R_{8b}$ und $R_{9a}$, $R_{9b}$ die für $R_{3a}$ und $R_{3b}$ angegebene Bedeutung haben; $R_{10}$ Wasserstoff oder eine $NH_2$-Schutzgruppe bedeutet und

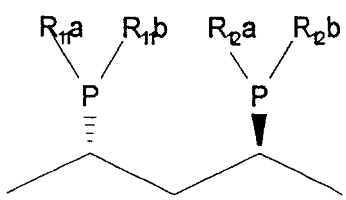

VIII

worin $R_{11a}$, $R_{11b}$ und $R_{12a}$, $R_{12b}$ die für $R_{3a}$ und $R_{3b}$ angegebene Bedeutung haben.

**2.** Verfahren nach Patentanspruch 1, dadurch gekennzeichnet, dass als auf eine bekannte Weise abspaltbare Gruppe für $R_1$ eine 1-Phenyl-($C_1$-$C_6$)-alkylgruppe oder eine 1-Naphthyl-($C_1$-$C_6$)-alkylgruppe eingesetzt wird, wobei die aromatischen Kerne der jeweiligen Gruppen gegebenenfalls mit einem oder mehreren Substituenten aus der Reihe ($C_1$-$C_6$)-Alkyl, ($C_1$-$C_6$)-Alkoxy, Hydroxy, Halogen, Amino, ($C_1$-$C_6$)-Alkylamino oder ($C_1$-$C_6$)-Dialkylamino substituiert sind.

**3.** Verfahren nach Patentanspruch 1 oder 2, dadurch gekennzeichnet, dass als auf bekannte Weise abspaltbare Gruppe für $R_2$ eine ($C_1$-$C_6$)-Alkanoylgruppe, eine ($C_1$-$C_6$)-Alkoxy-($C_1$-$C_6$)-alkylgruppe, eine ($C_1$-$C_6$)-Alkoxycarbonylgruppe, eine Aroylgruppe oder eine Benzylgruppe steht, wobei die aromatischen Kerne der jeweiligen Gruppen gegebenenfalls mit einem oder mehreren Substituenten aus der Reihe ($C_1$-$C_6$)-Alkyl, ($C_1$-$C_6$)-Alkoxy, Hydroxy, Halogen, Amino, ($C_1$-$C_6$)-Alkylamino oder ($C_1$-$C_6$)-Dialkylamino substituiert sind.

4. Verfahren nach einem oder mehreren der Patentansprüche 1 bis 3, dadurch gekennzeichnet, dass ein chiraler Phosphin-Ligand ausgewählt aus den Verbindungen der allgemeinen Formel V oder VI Anwendung finden.

5. Verfahren nach einem der Patentansprüche 1 bis 4 dadurch gekennzeichnet, dass als chiraler Phosphin-Ligand (R)-(S)-PPF-Pcy2 (allg. Formel V mit $R_{3a}$, $R_{3b}$ = Cyclohexyl und $R_{4a}$, $R_{4b}$ = Phenyl) oder (R)-(S)-PPF-PtBu2 (allg. Formel V mit $R_{3a}$, $R_{3b}$ = t-Butyl und $R_{4a}$, $R_{4b}$ = Phenyl) oder (4R, 5R)-MOD-DIOP (allg. Formel VI mit $R_5$, $R_7$ = Methyl und $R_6$ = Methoxy) eingesetzt wird.

6. Verfahren nach einem oder mehreren der Patentansprüche 1 bis 5, dadurch gekennzeichnet, dass ein Rh-Komplex der allgemeinen Formel

Rh(0): $[Rh\ (L)\ A]_2$ III

oder

Rh(I): $[Rh\ (L)_2]B$ IV

worin L für zwei $C_2$-$C_{12}$ Olefine oder ein $C_5$-$C_{12}$ Dien steht, A ein Halogen und B ein Anion einer Sauerstoffsäure oder einer Komplexsäure darstellt, eingesetzt wird.

7. Verfahren nach einem oder mehreren der Patentansprüche 1 bis 6, dadurch gekennzeichnet, dass die Umsetzung bei einem Wasserstoffdruck von 1 bis 200 bar und einer Reaktionstemperatur von 25 °C bis 150 °C durchgeführt wird.

8. Verfahren nach einem oder mehreren der Patentansprüche 1 bis 7 dadurch gekennzeichnet, dass die Katalysatormenge, ausgedrückt als molares Verhältnis Furoimidazol zu Homogenkatalysator, im Bereich 100 : 1 und 10'000 : 1 liegt.

9. Verfahren nach einem oder mehreren der Patentansprüche 1 bis 8, dadurch gekennzeichnet, dass aprotische Lösungsmittel eingesetzt werden.

10. Verfahren nach Patentanspruch 9, dadurch gekennzeichnet, dass als aprotisches Lösungsmittel Toluol eingesetzt wird.